# EUROPEAN PATENT APPLICATION

(11) **EP 3 795 574 A1**
(43) Date of publication of application: **24.03.2021**
(21) Application number: 19197919.4
(22) Date of filing: 18.09.2019
(51) Int. Cl.: C07F 15/00, A61P 35/00, A61K 31/282

(54) **SYNTHESIS AND USAGE OF 1,2-DIAMINODIAMANTANE PLATINUM(II) COMPLEXES**

(71) Applicant: Justus-Liebig-Universität Gießen, 35390 Gießen (DE)
(72) Inventor: Schreiner, Peter R., 35435 Wettenberg (DE); Fokin, Andrey A., 35398 Gießen (UA); Bakhonsky, Vladyslav V., 35392 Gießen (UA)
(74) Representative: Stumpf, Peter

(57) **Abstract**

A new bulky lipophilic chiral ligand based on 1,2-diaminodiamantane is revealed in both of its enantiomeric forms for the preparation of novel platinum(II) complexes. The R,R-enantiomer shows increased efficacy as compared to cisplatin. The binding of diamondoid-based platinum complexes to nucleotides occurs at similar or faster rate for both isomers compared to cisplatin so that the herein revealed platinum(II) complexes are compounds with high anti-cancerogenic activity .

## Description

### Field of the invention

The invention is about the synthesis of 1,2-diaminodiamantane platinum(II) complexes and the usage of either 1,2-diaminodiamantane platinum(II) complexes themselves or of compositions containing 1,2-diaminodiamantane platinum(II) complexes as medicament for use in the treatment of cancer of humans and/or mammals.

### Background of the technology

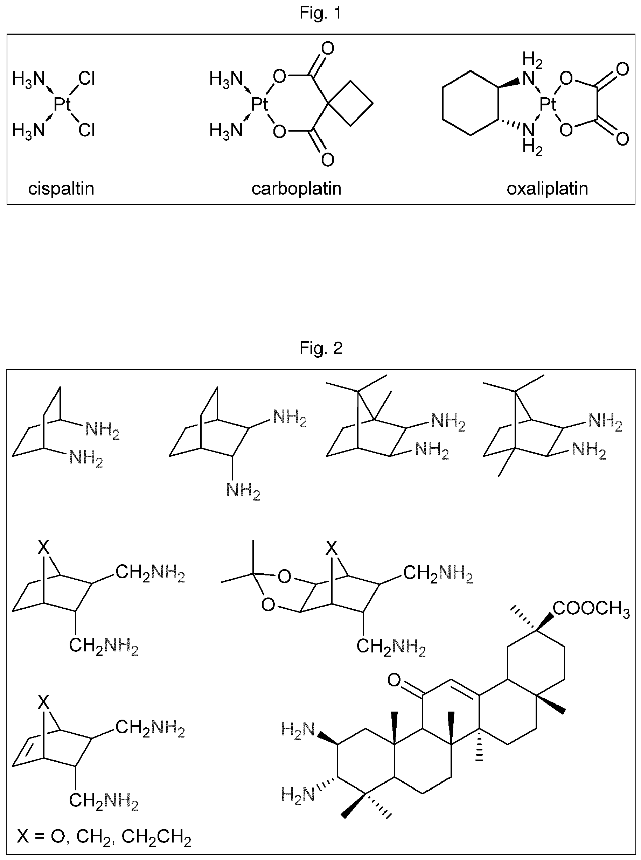

Cisplatin [*cis*-dichlorodiamineplatinum(II)] (Fig. 1), first tested in 1968, was approved worldwide in 1978 after being accidentally discovered to have antiproliferative activity by Rosenberg during electrochemical treatment of living cells using platinum electrodes. This discovery dramatically changed cancer treatment regimens with platinum-based drugs being incorporated in >50% of formulations as effective agents by themselves or in combination with other antineoplastic medication for a wide variety of cancer cell types. Cisplatin is still the preferred drug for ovarian and testicular cancer providing 90% cure rate for the latter. The mechanism of action of cisplatin and its analogues with the general formula [PtX₂(RNH₂)₂] can be rationalized by hydrolysis of the diamine complexes inside cancer cells producing the charged [Pt(NH₃)₂(H₂O)₂]²⁺ complex that quickly loses water and forms inter- and intrastrand DNA cross-links. The majority of cross-links are guanosine-guanosine d(GpG) and, to lesser extent, adenosine-guanosine d(ApG) intrastrand cross-links formed by platinum diamine complexes and adjacent DNA purine bases. The resulting platinum adducts interfere with DNA replication triggering cell apoptosis. Besides beneficial cytotoxicity used to target cancer cells, cisplatin treatment has drawbacks such as severe nephrotoxicity, being potentially lethal for patients without modern hydration techniques, dose-limiting peripheral neurotoxicity, gastrointestinal toxicity, ototoxicity, and hematological toxicity. Furthermore, it quickly became evident that numerous cancer cell types have intrinsic or acquired resistance towards cisplatin. In an attempt to combat the shortcomings of cisplatin, carboplatin was developed. This second-generation platinum agent has chlorine ligands substituted by a bidentate cyclohexane dicarboxylate moiety. Carboplatin has a greatly reduced toxicity profile allowing to adjust the active dosage to a particular patient, although it has decreased anticancer activity requiring 20-40 fold increase in concentration to achieve the same effect. Despite being actively used in treatment of ovarian cancer, the dose-limiting mye-losuppression (thrombocytopenia and to lesser extent neutropenia and anemia) still remains a significant drawback. Carboplatin has the same mechanism of action inside the cell as cisplatin. Consequently, numerous cancer cell types known to acquire cisplatin resistance exhibit cross-resistance to carboplatin as well.

The third-generation platinum reagents was developed to further counter cisplatin resistance. Oxaliplatin, the most recent worldwide-approved anticancer drug, displays a bidentate oxalate moiety instead of the two chloride anions in cisplatin, as well as amino ligands derived from (1*R*,2*R*)-1,2-diaminocyclohexane [(*R*,*R*)-DACH]. The bigger and more lipophilic 1,2-diaminocyclohexane changes the cytotoxicity profile and provides somewhat limited cross-resistance to cisplatin that is linked to the steric properties of the DACH adduct that hinders the DNA repair mechanism within tumor cells. Differences in activity manifest themselves in the effectiveness of oxaliplatin in treatment of colorectal cancer, which is insensitive to cisplatin and carboplatin.

New generations of anticancer drugs with diverse cytotoxicity profiles require different diamine carrier ligands that are responsible for interactions with DNA inside cancer cells. The lipophilicity of these ligands increase absorption and accumulation of the platinum complex within cell while decreasing the requirement for renal excretion, thus reducing toxic side effects. *Trans-1,2-*diaminocyclohexane is the most popular diamine pharmacophore, although other hydrocarbon backbones receive increasing attention. One of them is kiteplatin ([PtCl₂(*cis*-1,4-DACH)]), first synthesized in 1994, which incorporates a 1,2-DACH isomer; it lacks cross-resistance with already approved anticancer drugs. More bulky and lipophilic 2,3-diaminobicyclo[2.2.1]heptane, 2,3-diaminobicyclo[2.2.1]heptane, camphor-1,2-diamine, and their analogues (Fig. 2) were recently utilized as carrier ligands of which some exhibited promising antiproliferative properties in platinum complexes when paired with appropriate ligands.

The decreased anticancer activity of, e.g., carboplatin, compared to cisplatin is a major disadvantage within the current state of the art as well as the existing cross-resistance between cisplatin and carboplatin.

Using diamino-ligands with two adjacent tertiary amino-substituents as diamino ligands in platinum complexes is neither known nor suggested within the state of the art. This origininates from the synthetic difficulty in the preparation of highly lipophilic, adjacent tertiary amines, in particular, in combination with a cage hydrocarbon structure that prevents backside reactions at reactive synthons.

### Content of the invention

It is therefore the aim of the present invention to provide new platinum complexes comprising diamino ligands with two adjacent tertiary amino-substituents, their synthesis/production/manufacturing and their usage for cancer treatment. This task is solved by characterising the technical features of claims 1 through 3; the dependent claims reveal additional preferred embodiments of the invention.

Surprisingly it was found that - for example - 1,2-diaminodiamantane is a viable and advantageous alternative to known lipophilic diamine pharmacophores and can be used for synthesizing compounds according to the invention. Our recent work on diamantane functionalization provides direct access to previously unknown 1,2-disubstituted diamondoids and 1,2-diaminodiamantane in particular. The bulky diamantane backbone provides high lipophilicity in a compact and extraordinarily stable lattice. Being conformationally rigid, 1,2-diaminodiamantane may present significant steric and kinetic hindrance to the DNA repair mechanism, which may further increase the efficacy of its platinum-DNA adduct. We surprisingly found that the desired lipophilicity of platinum complexes for medical applications can advantageously be achieved in combination with the very compact backbone of the 1,2-diaminodiamantane ligand. Therefore the platinum complexes according to the invention now for the first time combine two different characteristics for the medical applicability of platinum complexes for treating cancer, which were formerly impossible to combine:
1.) being lipophilic and voluminous on one hand in order to provide effective steric and kinetic hindrance to the DNA repair mechanism and
2.) being small enough for more easily intruding into the major groove and/or the minor groove of the DNA double helix for chemically interacting with the DNA.
The two available stereoisomers of 1,2-diaminodiamantane are the *R,R* and *S,S* enantiomers that introduce chirality into their platinum complexes, which is known to play a major role in platinum drug efficacy.

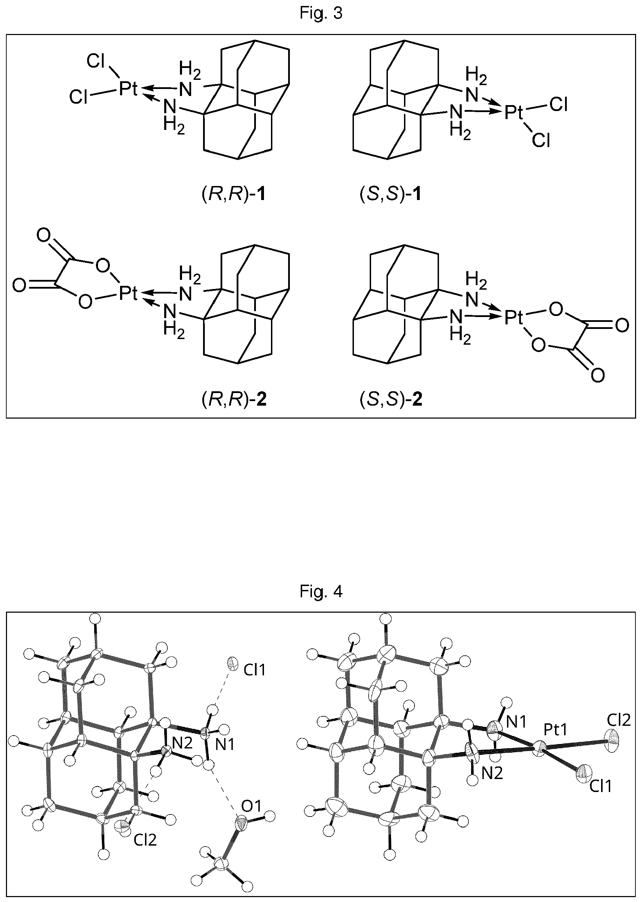

Herein two diamantane based platinum complexes are exemplarily synthesized in both of their enantiopure forms (Fig. 3) and tested in cancer cell assays. The scope of the invention or its equivalents is not confined to these two examples. Especially the usage of substituted 1,2-diamino-diamantanes lies well within the scope of the invention or its equivalents. The (*R*,*R*)-**2** and (*S*,*S*)-**2** complexes were found to have very low solubility and were not yet used in activity studies. Although these complexes (termed *diaplatins)* generally exhibit decreased solubility compared to cisplatin, we were able to grow a crystal of (S,S)-**1** and compare its geometry to the structures of 1,2-diaminocyclohexanedichloroplatinum(II) [PtCl₂(1,2-DACH)], and cisplatin. The diaplatin enantiomers were tested for their cytotoxicity via a cell viability assay conducted on the human ovarian cancer cell line A2750 and its cisplatin-resistant variant A2780cis. (*R*,*R*)-**1** displays higher cytotoxicity than cisplatin for both cell lines, while (S,S)-**1** shows lower or similar activity as compared to cisplatin, which is in agreement with the known reactivity of oxaliplatin comprising (R,R)-DACH and its *S*,*S*-diamine-based counterpart. We evaluated GMP and dGMP binding rates for both (*R*,*R*)-**1** and (*S*,*S*)-**1** complexes using NMR spectroscopy. Despite the increased steric demand, charged diamantanedi-aminoplatinum complexes have binding constants comparable to cisplatin. *(R,R)-1* shows increased activity and cytotoxicity compared to cisplatin, thereby benefiting from its bulky backbone with inherent chirality.

### Platinum complexes synthesis

For the synthesis of diaplatin complexes **1** and **2** we decided to exemplarily utilize silver-free methods to prevent microscopic concentrations of silver from contaminating the samples. Silver is a potent cytotoxic agent that requires multiple crystallizations in addition to other purification procedures to avoid its influence on the cytotoxicity profile of platinum samples. To prepare (*R*,*R*)-**1** and (*S*,*S*)-**1**, the pure 1,2-diaminodiamantane enantiomers were reacted with K₂PtCl₄. The reaction mixture was stirred for three days protected from light, followed by filtration. The (*R*,*R*)-**2** and (*S*,*S*)-**2** oxalate complexes, K₂[Pt(ox)₂] prepared from K₂PtCl₄ and potassium oxalate, were reacted with the 1,2-diaminodiamantane enantiomers in a similar fashion. This procedure gives diamantane-based platinum complexes in good yields (Scheme 1).

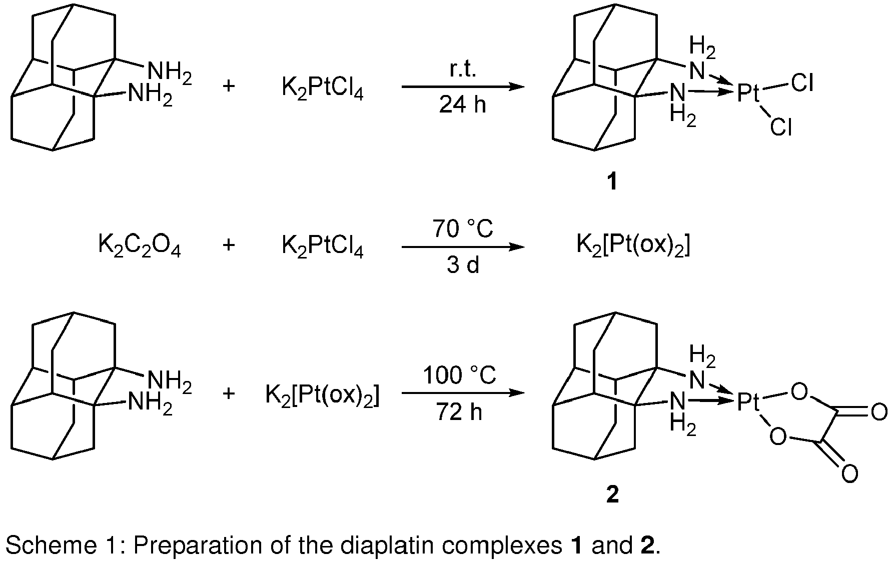

The complexes are yellow, which is common for this class of platinum structures, and they have decreased solubility compared to cisplatin. Products (*R*,*R*)-**2** and (*S*,*S*)-**2** are insoluble in most common solvents including *N,N-*dimethylformamide (DMF), known to dissolve platinum complexes. However, 1 displays good solubility in DMF and is somewhat soluble in methanol. After initial tests (*R*,*R*)-**2** and (*S*,*S*)-**2** were discarded as not soluble enough to reach active concentrations for cytotoxicity assays.

### Structure analysis

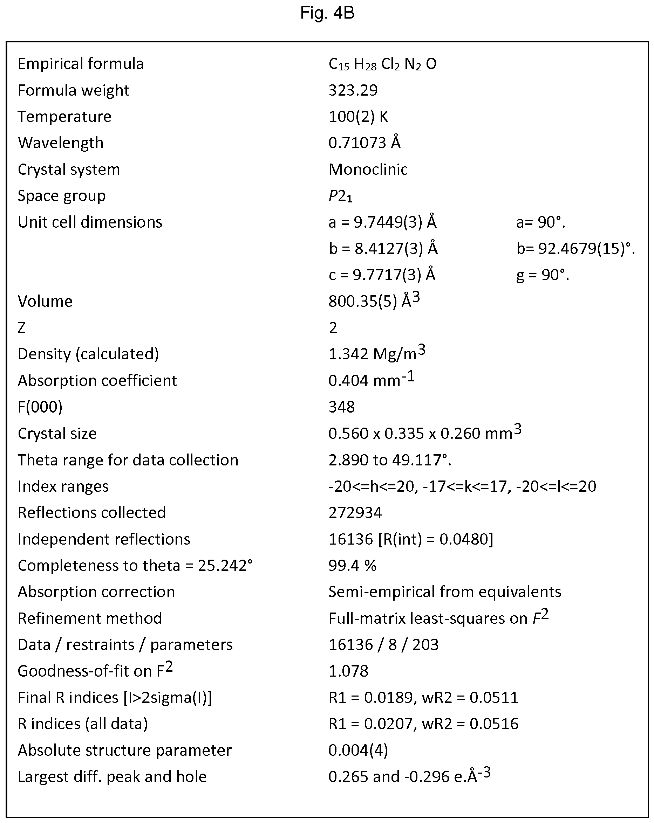

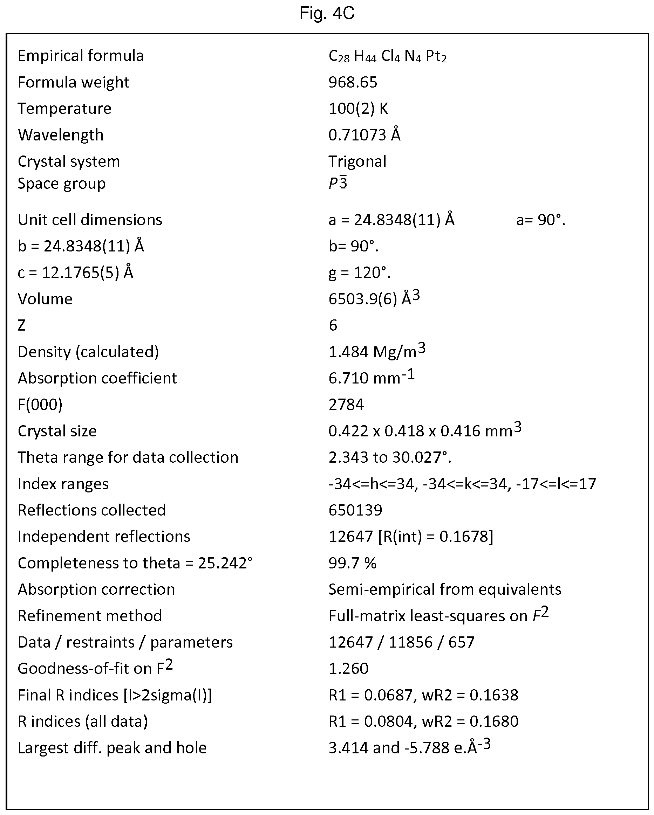

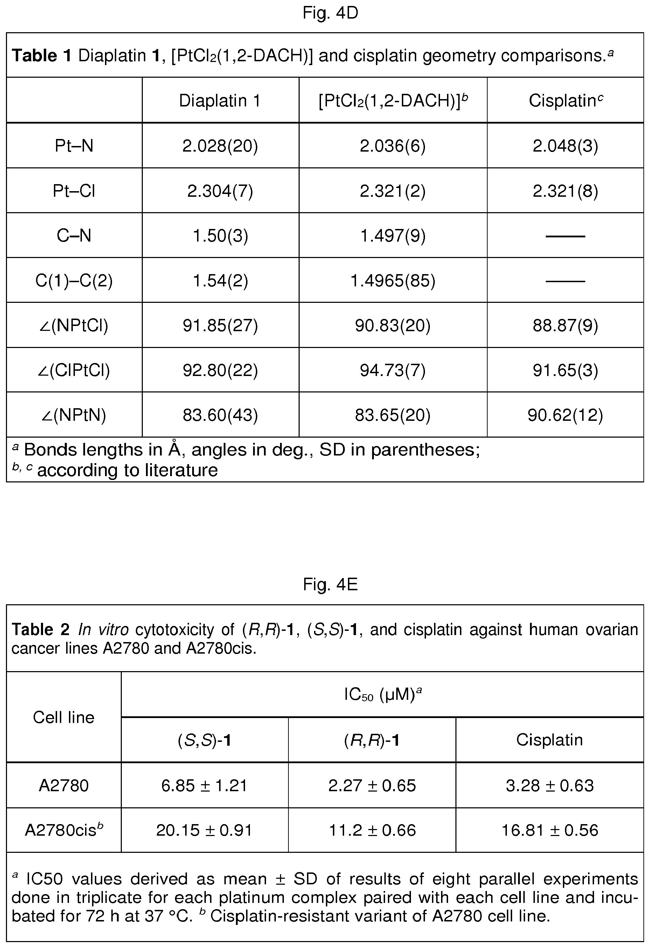

Crystallization of the 1,2-diaminodiamantane dihydrochloride from methanol gave crystals suitable for analysis. The solved structure (Fig. 4) shows an absolute configuration of (S,S), determined via anomalous dispersion, with a high degree of purity reflected in Flack parameter being close to zero (Fig. 4B). Using a two-phase diffusion technique with DMF as the primary solvent and diethyl ether as the second phase, we were able to grow a crystal of (*S*,*S*)-**1** and obtain its crystal structure (Fig. 4). The (*S*,*S*)-**1** crystal resolved in the centrosymmetric space group P3 despite containing a chiral diamine moiety (Fig. 4C). As racemization of 1,2-diaminodiamantane would require bond cleavage, it is obvious to the person skilled in the art that the platinum complex preserves the absolute configuration of the diamine ligand. We compared the crystal structure of **1**, [PtCl₂(1,2-DACH)], and cisplatin (Table 1, Fig. 4D). Complex **1** has a longer C1-C2 bond than the 1,2-DACH complex and has slightly shorter Pt-Cl bonds as compared to the other two complexes. The N-Pt-Cl angle of **1** is the largest of the three, while the Cl-Pt-Cl angle is slightly smaller than the corresponding angle in [PtCl₂(1,2-DACH)]. Overall, surprisingly the geometry of **1** is very close to the geometries of [PtCl₂(1,2-DACH)] and cisplatin with a few differences imposed by the rigid diamondoid moiety, although **1** exerts a by far more rigid structure than 1,2-DACH, caused by the rigid cage structure and the two directly adjacent tertiary amino groups attached to it.

### Cellular viability assay

The platinum complexes were tested against the human ovarian cancer cell line A2780 and its cisplatin-resistant variant A2780cis. Structures (*R*,*R*)-**1**, (*S*,*S*)-**1**, and cisplatin were introduced to cell cultures in ten different concentrations (Figure 3) and incubated for 72 h at 37 °C. We used standard procedures for adding a fluorescent dye to cell wells and, using spectrophotometry, we gathered dose-response data to determine the IC₅₀ values (Table 2, Fig. 4E). (*R*,*R*)-**1** shows superior cytotoxicity compared to cisplatin for both cancer cell lines while (*S*,*S*)-**1** has the lowest efficacy. The difference in activity between the diaplatin stereoisomers is in line with previously observed pattern for oxaliplatin, which contains the (*R*,*R*)-DACH fragment. The difference in reactivity of oxaliplatin and its isomer is attributed to a more favorable configuration of the resulting DNA cross-link, according to literature. The cisplatin-resistant cell line A2780cis shows a notable decrease in response to platinum complexes in the same range of concentrations, although (*R*,*R*)-**1** retains its superior activity compared to cisplatin. Tested efficacy of simplest 1,2-diaminodiamantane platinum complexes can be further enhanced by substituting the chloride groups for other leaving ligands, which can enhance solubility, tune the platinum complex for specific cancer cells lines, and decrease overall toxicity for the human body without leaving the scope of the invention, as is obvious to the person skilled in the art.

### Nucleotide binding experiments

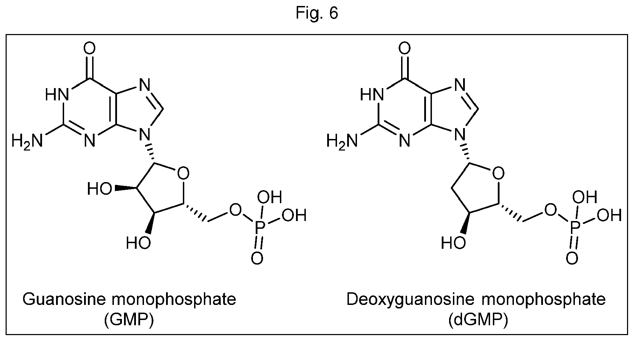

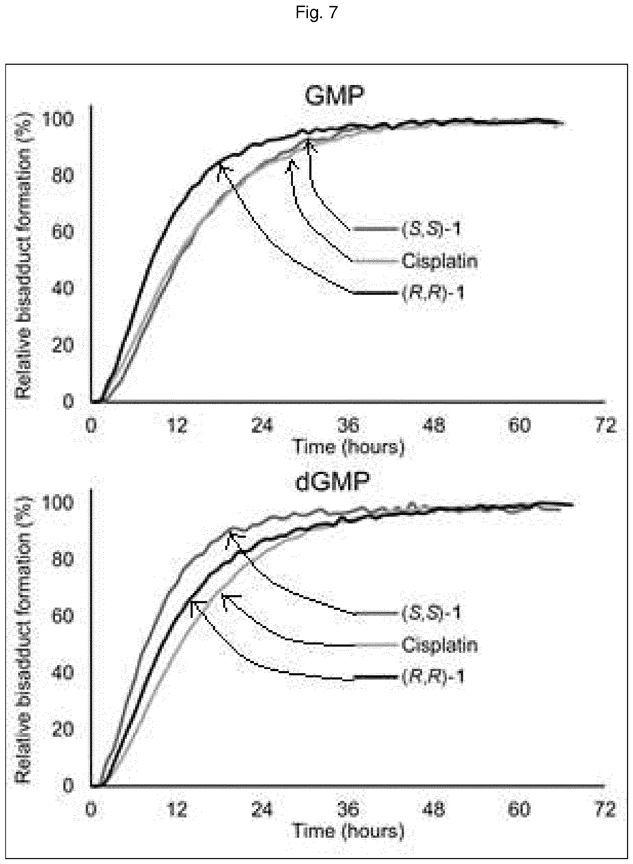

Formation of DNA cross-links is the crucial part of the platinum cytotoxicity mechanism. To study diaplatin complex **1** activity on nucleotides in comparison to cisplatin we chose guanosine monophosphate (GMP) and deoxyguanosine monophosphate (dGMP) as substrates (Fig 6). The reaction between platinum complexes and GMP or dGMP causes substitution of their leaving group ligands with nucleotide forming monoadducts that quickly lose a second leaving ligand forming bisadducts. The N7 atom of the purine fragment is known to be the preferred site of attachment to platinum, thus causing easily observable downfield NMR shifts of the adjacent H8 atom. The collected ¹H NMR data during incubation of the reaction mixtures provide time-dependent changes of the H8 atom integral, thereby providing insights into the binding rates between the studied complexes (Fig 7). Surprisingly both nucleotides form bisadducts faster with (*R*,*R*)**-1** than with cisplatin despite the bulky diamantane backbone. The (*S*,*S*)-**1** bisadduct with GMP has a rate of formation similar to cisplatin, but binding to deoxyguanosine proceeds faster than for (*R*,*R*)-**1** and cisplatin. Overall, diaplatin **1** shows noticeably faster reaction towards bisadduct formation despite greatly increased steric requirements posed by the diamantane moiety.

1,2-Diaminodiamantane is revealed herein to be a viable non-leaving ligand for platinum-based anticancer drugs. The inventors are able to prepare (*R*,*R*)-**1** and (*S*,*S*)-**1** featuring - for example - chloride ligands. The analogous platinum complexes with oxalate moieties were synthesized as additional examples. The crystallographic data confirm the absolute configurations of the newly prepared complexes. A cytotoxic assay demonstrates the superiority of (*R,R*)-**1** relative to cisplatin on both the human ovarian cancer cell line A2780 and its cisplatin-resistant variant A2780cis. Complex (*S*,*S*)-**1** was found to be less potent than cisplatin on either cell line. This further demonstrated the difference in reactivity caused by the chiral diamine backbone interactions with DNA. Similar to oxaliplatin, which contains the *R,R*-enantiomer of *trans*-1,2-diaminocyclohexane as the diamine ligand, (*R*,*R*)-1,2-diaminodiamantane yields the more potent cytotoxic complex. Nucleotide binding studies of the diaplatin enantiomers show activities similar to other platinum (II) complexes. Despite the increased steric demand, both GMP and dGMP surprisingly bind faster to (*R*,*R*)-**1** than to cisplatin. The (*S*,*S*)**-1** binding kinetics towards GMP are similar to cisplatin, but in case of dGMP the rate is higher than that of (*R*,*R*)-**1** or cisplatin. 1,2-Diaminodiamantane surprisingly provides an opportunity to utilize a pharmacophore with an unusual set of properties. The simplest platinum (II) complex based on this diamantane backbone already proves to be more potent than cisplatin on the studied cell lines while being as fast at binding with nucleotides as cisplatin despite considerably increased bulk. Furthermore, the straightforward diaplatin synthesis allows simple modifications of the ligands as a way to improve solubility in addition to augmenting its pharmacokinetics.

It is well known to the person skilled in the art, that the exemplarily performed experiments as shown in the following paragraphs do not confine the scope of the invention to these examples.

It is also well known to the person skilled in the art that by substituting the diamantane moiety of the platinum complexes the anti-proliferating effectiveness, solubility, stability etc. can be modified without leaving the scope of the invention or its equivalents. Therefore the invention comprises platinum complexes with 1,2-diaminodiamantane ligands of formula **I**:

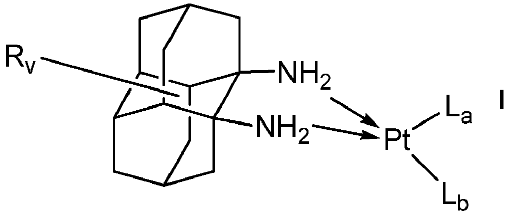

Thus the at least one substituent -Rᵥ of the substituted 1,2-diaminodiamantane moiety of the platinum complexes is most preferredly be chosen from the list comprising the substituents -H, -F, -Cl, -Br-, -I, methyl, ethyl, propyl, isopropyl, n-butyl, sec-butyl, tert-butyl, phenyl, -OH, -OMe, -OEt, -NH₂ or any other suitable substituent.

To the person skilled in the art it is well known that the efficacy of an active ingredient of a medicament may often be improved, i.e. adapted regarding its efficacy, bioavailability, biodistribution, stability and/or compatibility, via chemical modification, e.g. etherification, esterification, sulfonation, substitution, elimination, cyclisation etc. For example, it is well known that fluorination, i.e. substitution of hydrogen atoms by fluorine atoms is prolonging the time span the active ingredient stays within the body of the patient, thus intensifying the efficacy. Therefore it is obvious to the person skilled in the art that any derivative of 1,2-diaminodiamantane as ligand for platinum complexes that shows better anti-proliferation activity towards cancer cells and/or less unwanted side effects lies within the scope of the invention. A sample list of further possible substituents -Rᵥ comprises -CF₃, -C₂F₅, -C₃F₇, -alkyl, -cycloalkyl, -aryl, -heteroaryl, -acyl, -SOnRf, -SOnH, -POmRf, -POmH, -SOzN(Rf)2; -POm(NRf)x, -SOz(NRf)y, -B(ORf)2, -OB(ORf)2, -B(ORf)(NRf), -B(NRf)2, -OB(ORf)(NRf), -OB(NRf)2, substituted -aryl, substituted -heteroaryl, -alkylaryl, -alkylheteroaryl, -OH, -phenyl, -NH₂, -NO₂, -OR_{f}, -O-COR_{f}, -COR_{f}, -N(R_{f})₂, -NR_{f}COR_{f}, -O-CSR_{f}, -CSR_{f}, -NR_{f}CSR_{f}, -O-CSeR_{f}, -CSeR_{f}, -NR_{f}CSeR_{f}, -O-CN(R_{f})R_{f}, -CN(R_{f})R_{f}, -NR_{f}CN(R_{f})R_{f}, -N(R_{f})SO₂R_{f}, -N(R_{f})SO₂N(R_{f})₂, -SSR_{f}, -SR_{f}, -SOₓR_{f}, -SOₙN(R_{f})₂, -nitril, -amidine, -guanidine, -COOR_{f}, -CON(R_{f})₂, -CON(R_{f})N(R_{f})₂, -COSR_{f}, -CSOR_{f}, -CSSR_{f}, -NR_{f}COR_{f}, -N(R_{f})N(R_{f})₂, -NHR_{f}, -NR_{f}R_{g}, -CONR_{f}R_{g}, -NHCOR_{f}, -NR_{f}COR_{g}, -CSR_{f}, -CSNHR_{f}, -CSNR_{f}R_{g}, -CSR_{f}, -OCSR_{f}, -NR_{f}CSR_{f}, -NR_{f}CSR_{g}, -COSR_{f}, -CSNR_{f}N(R_{f})₂, -CONR_{f}N(R_{f})₂, -NR_{f}N(R_{f})₂.

Substituents R_{f} and R_{g} are independently from each other chosen from the list comprising -H, -alkyl, -cycloalkyl, -aryl, -heteroaryl, substituted -aryl, substituted -heteroaryl, -alkylaryl, -alkylheteroaryl.

The substituents of the substituted -aryl and substituted -heteroaryl groups are chosen from the list comprising -F, -Cl, -Br, -OH, -phenyl, -NH₂, -NO₂, -OR_{f}, -O-COR_{f}, -COR_{f}, -N(R_{f})₂, -NR_{f}COR_{f}, -O-CSR_{f}, -CSR_{f}, -NR_{f}CSR_{f}, -O-CSeR_{f}, -CSeR_{f}, -NR_{f}CSeR_{f}, -O-CN(R_{f})R_{f}, -CN(R_{f})R_{f}, -NR_{f}CN(R_{f})R_{f}, -N(R_{f})SO₂R_{f}, -N(R_{f})SO₂N(R_{f})₂, -SSR_{f}, -SR_{f}, -SOₓR_{f}, -SOₙN(R_{f})₂, -nitrile, -amidine, -guanidine, -COOR_{f}, -CON(R_{f})₂, -CON(R_{f})N(R_{f})₂, -COSR_{f}, -CSOR_{f}, -CSSR_{f}, -NR_{f}COR_{f}, -N(Rf)N(Rf)2 .

The formular indices "x" and "y" can adopt values from 1 to 2, formular index "n" can adopt values from 0 to 4 and formular index "m" can adopt values from 1 to 4.

This list is not limiting the scope of the invention, 1,2-diaminodiamantane may be multiply substituted, the substituents are being chosen independently from each other and may be attached to any part of the base molecule.

It is well known to the person skilled in the art that the combination of compounds with anticancer activity, e.g. cisplatin, can be advantageously combined with other anti-proliferative compounds in order to enhance the antitumor-activity, due to synergistic effects. Therefore it lies well within the scope of the invention or its equivalents to combine a 1,2-diaminodiamantane platinum(II) complex with other anti-proliferative compounds and thus using the synergistic effect of the combination of, e.g., two or more anti-proliferative compounds with different mechanisms of anti-proliferative activity.

The invention can be summarized as follows:

The invention comprises one or more compounds according to formula **I,** 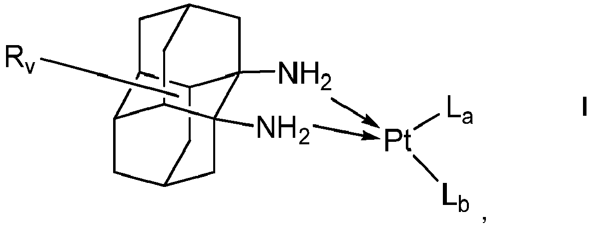 characterized in that
- the diamantane core-structure is substituted with at least one substituent Rᵥ, independently from each other chosen from the list comprising the substituents -H, -F, -Cl, -Br-, -I, methyl, ethyl, propyl, isopropyl, n-butyl, sec-butyl, tert-butyl, phenyl, -OH, -OMe, -OEt, -NH₂;
- the ligands Lₐ and L_{b} are independently chosen from the list comprising the ligands -F, -Cl, -Br, -I, -OH, -OR
   or
   the ligands Lₐ and L_{b} are together forming a bidentate/bivalent ligand of the type -X-(CH₂)s-Y- whereat
   X and Y are independently from each other chosen from the substituents -CO₂H, -CNR(OH), -CS(OH) or their deprotonated forms, and the index s adopts a value of 0, 1, 2, 3 or 4.

The invention further comprises a process for the production of a compound according to formula **I,** 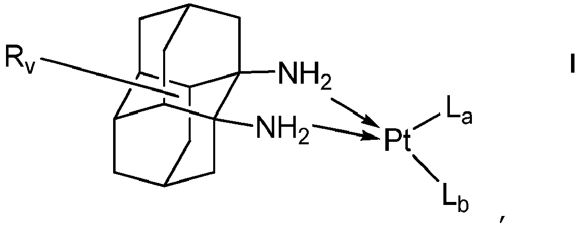 characterized in that the process comprises the following steps:
a) preparing a solution
   i) of a salt of formula M₂PtX₄
      or
   ii) of a salt of formula M₂[Pt(L)₂]
      whereat
      M is at least one metal independently chosen from the alkali metals Li, Na, K, Rb, Cs
      and
      iii) X is at least one halogen, independently chosen from the list of halogens comprising F, Cl, Br, I
         or
      iv) L is a bidentate ligand of the type -X-(CH₂)s-Y-, whereat
         X and Y are independently from each other chosen from the substituents -CO₂⁻-, -CNR(O)⁻, -CS(O)⁻,
         and the index s adopts a value of 0, 1, 2, 3 or 4;
b) adding 1,2-diaminodiamantane or a solution of 1,2-diaminodiamantane to the solution according to step a) whereby a reaction mixture is formed;
c) stirring the reaction mixture of step b) whereby a precipitate is formed;
d) removing the precipitate from step c) and purifying it in order to get the pure compound according to formula **I.**

The invention further comprises a medicament comprising a therapeutic effective amount of a compound according to formular **I** as is described above for use in the treatment of cancer of humans and/or mammals.

### Detailed embodiments of the invention

### Synthesis of platinum complexes

Chemicals, solvents, and reagents for biological assays were of analytical or equivalent grade and were used as received. The synthesis and purification of the ligand 1,2-diaminodiamantane is well known to the person skilled in the art.

### cis-[1,2-diaminodiamantane]dichlorideplatinum(II) (1):

1,2-Diaminodiamantane (0.1 g, 0.46 mmol) was added to a solution of K₂PtCl₄ (0.19 g, 0.46 mmol) in water (2 mL) and the resulting mixture was stirred for 24 h at rt. A yellow precipitate formed and was filtered, washed with acetone, recrystallized from a water/DMF mixture, and dried under reduced pressure at 40 °C for 24 h. We obtained yellow crystals of **1** (0.085 g) in 37% yield. Mp 314 °C (dec.). ¹H NMR (300 MHz, DMF-d7): δ 5.18 - 4.84 (m, 4H), 2.78 - 2.66 (m, 2H), 2.65 - 2.51 (m, 2H), 2.04 - 1.93 (m, 4H), 1.77 - 1.66 (m, 4H), 1.66 - 1.55 (m, 4H), 1.47 - 1.35 (m, 2H). ¹³C NMR (75 MHz, DMF-d7): δ 64.5 (C), 43.5 (CH), 40.2 (CH), 38.2 (CH₂), 38.0 (CH₂), 31.7 (CH₂), 28.8 (CH). ¹⁹⁵Pt (64 MHz, DMF-d7): δ 2251. HRMS (ESI): calcd for C₁₄H₂₂N₂Cl₂NaPt⁺ [M+Na]⁺ = 506.0700 found 506.0702.

### cis-[1,2-diaminodiamantane]oxalatoplatinum(II) (2):

A solution of 1,2-diaminodiamantane (0.1 g, 0.46 mmol) in isopropanol (5 mL) was added to a solution of K₂[Pt(ox)₂]·2H₂O (0.187 g, 0.41 mmol) in water (5 mL) preheated to 50 °C. The resulting mixture was stirred at 70 °C for 72 h in the dark. The precipitated yellow-grey powder was filtered, washed with water and acetone, and dried under reduced pressure. We obtained yellow-grey crystals of **2** (0.06 g) in 29% yield. Mp 267 °C (dec.). IR vₘₐₓ/cm⁻¹: 2913*br*, 1694*s*, 1667*s*, 1598*w*, 1357*s*, 818*s*, 561*w*, 459*w*. Anal. Calcd for C₁₆H₂₂N₂O₄Pt: C, 38.32; H, 4.42; N, 5.59. Found: C, 37.63; H, 4.42; N, 5.23.

### Crystallographic structure measurements

Crystallographic data for 1,2-diaminodiamantane dihydrochloride and (*S*,*S*)-**1** were collected at 100 K using *ϕ*- and *ω*-scans on a BRUKER D8 Venture system equipped with dual IµS microfocus sources, a PHOTON100 detector and an OXFORD CRYOSYSTEMS 700 low temperature system. Mo-*Kα* radiation with a wavelength of 0.71073 Å and a collimating Quazar multilayer mirror were used. Semi-empirical absorption corrections from equivalents were applied using SA-DABS-2016/2 the space groups were determined by systematic absences using XPREP and the structures were solved by direct methods using SHELXT. Refinement for all structures was performed against F² on all data by full-matrix least squares using SHELXL. All non-hydrogen atoms were refined anisotropically and C-H hydrogen atoms were positioned at geometrically calculated positions and refined using a riding model. The isotropic displacement parameters of all hydrogen atoms were fixed to 1.2x or 1.5x (CH₃ and OH hydrogens) the *U_{eq}* value of the atoms they are linked to.

The unit cell of (S,S)-1,2-diaminodiamantane (cf. Fig. 4B) was determined using 9978 reflections and the structure was solved in the monoclinic space group P2₁. The asymmetric unit contains the cation, two chloride anions and a methanol molecule. The absolute configuration of the structure could be successfully determined utilizing the anomalous dispersion effect and using the improved Flack method with a Flack x of 0.004(4).

The unit cell of (*S*,*S*)-**1** (cf. Fig. 4C) was determined using 9150 reflections and the structure was solved in the trigonal space group P3. The asymmetric unit contains besides heavily disordered solvent molecules two molecules of (*S*,*S*)-**1** and it shows whole molecule disorder for both molecules. The whole molecule disorder may be a reason that the structure was only refinable in the centrosymmetric space group. The disorder was modelled with the help of 1,2 and 1,3 distance similarity restraints, similarity restrains on all anisotropic atomic displacement parameters and advanced rigid bond restraints. Additionally some atoms were set to have the same anisotropic atomic displacement parameters. The SQUEEZE-method as implemented in Platon was used to include a model for the disordered solvent molecules. The obtained model was added as fab-file to the refinement using SHELXL. SQUEEZE found one independent void with disordered solvent, located at 0.0 0.0 0.0, with a volume of 2187 Å³. The equivalent of 1378 electrons were identified in the void.

### Cellular viability assay

The human ovarian cancer cell line A2780 (ECACC 93112519) and its cisplatin-resistant variant A2780cis (ECACC 93112517) were grown as a monolayer at 37 °C in an atmosphere containing 5% CO₂ in RPMI medium (Gibco BRLTM, Invitro-gen Corporation, Netherlands) with 10% fetal bovine serum (Hyclone, Perbio Science, Netherlands), benzylpenicillin sodium salt (Penicillin G sodium salt, 100 units/mL; Dufecha, Biochemie BV, Netherlands), streptomycin (100 µg/mL; Dufecha, Biochemie BV, Netherlands), and 2 mM Glutamax x100 (Gibco BRLTM, Netherlands). The cells were seeded at 20000 cells cm⁻² in 96-well plates after transferring them with a micropipette from the source culture after trypsinization. Diaplatins and cisplatin as the standard were introduced as solutions in DMF. The concentration in the first well was 270 µM and 1% DMF for (*R*,*R*)-**1**, (*S*,*S*)-**1**, cisplatin and 1% DMF for the control experiment. In every following well, the concentration of the platinum complexes and DMF was progressively halved. Cells were incubated for 72 h at 37 °C in an atmosphere containing 5% CO₂. The cell viability was then analyzed by addition of resazurin sodium salt (Alamar Blue) to cell cultures followed by incubation for 6 h and fluorescence assay of the thus produced resorufin. The evaluation of cell survival was performed with an Epoch Microplate Spectrophotometer (BioTek Instruments) to measure absorbance at 570 nm. All conducted cytotoxicity experiments for (*R*,*R*)-**1**, (*S*,*S*)-**1**, cisplatin, and the control solution containing DMF were run eight times in parallel for each cell line and were done in triplicate. The cytotoxicities of the platinum complexes were calculated using non-linear regression analysis of dose-response data and expressed as half maximal inhibitory concentrations (IC₅₀). Statistical analysis was performed with GraphPad Prism 7.

### Nucleotide binding experiments

The rates of (*R*,*R*)-**1**, (*S*,*S*)-**1**, and cisplatin binding with guanosine monophosphate (GMP) and deoxyguanosine monophosphate (dGMP) nucleotides were studied using ¹H NMR spectroscopy. A platinum complex solution in DMF-d7 (0.27 mL, 6.9 mM) was reacted with GMP or dGMP in a molar ratio 1:3.5 introduced with a NaClO₄ solution in D₂O (0.33 mL, 100 mM). The reaction mixture was immediately transferred into an NMR tube and ¹H NMR data were collected every hour for 66 h at 37 °C. The pH was measured immediately after the reaction mixture was prepared and at the end of the reaction at rt.

### Description of the drawings

- Fig. 1:: Chemical structures of platinum anticancer drugs approved worldwide.
- Fig. 2:: Bulky diamine ligands known to be tested in various platinum complexes for antiproliferative activity.
- Fig. 3:: Exemplarily investigated diamondoid-based platinum complexes accord-ing to the invention.
- Fig. 4:: The crystal structures of (S,S)-1,2-diaminodiamantane dihydrochloride in a cell with a methanol molecule and of (S,S)-**1**. Ellipsoids drawn at 50% probability.
- Fig. 4B:: Crystal data and structure refinement for (S,S)-1,2-diaminodiamantane.
- Fig. 4C:: Crystal data and structure refinement for (S,S)-**1**.
- Fig. 4D:: Table 1: Diaplatin **1**, [PtCl₂(1,2-DACH)] and cisplatin geometry comparisons.
- Fig. 4E:: Table 2: In vitro cytotoxicity of (R,R)-**1**, (S,S)-**1**, and cisplatin against human ovarian cancer lines A2780 and A2780cis.

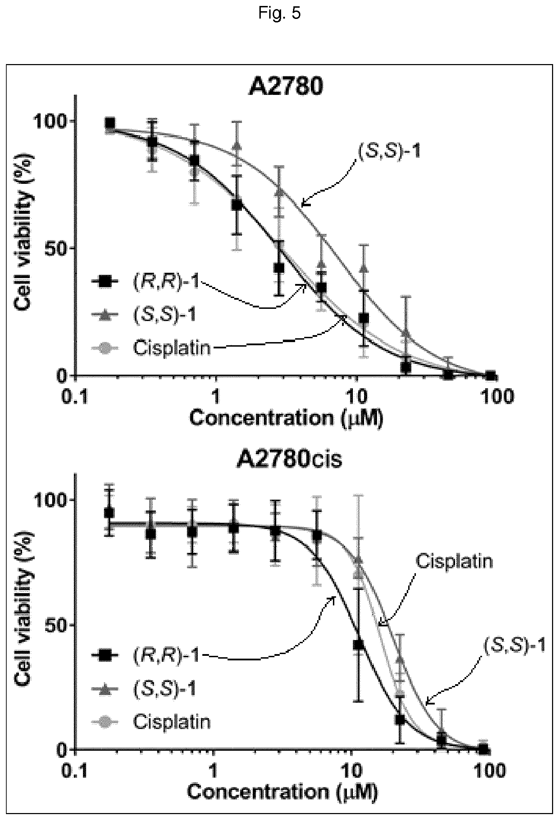

- Fig. 5:: Dose-response curves for (R,R)-**1**, (S,S)-**1**, and cisplatin on human ovarian cancer cell lines A2780 (upper graph) and A2780cis (lower graph).
- Fig. 6:: Structures of used nucleotide substrates.
- Fig. 7:: Rates of nucleotide-platinum bisadduct formations for GMP and dGMP.

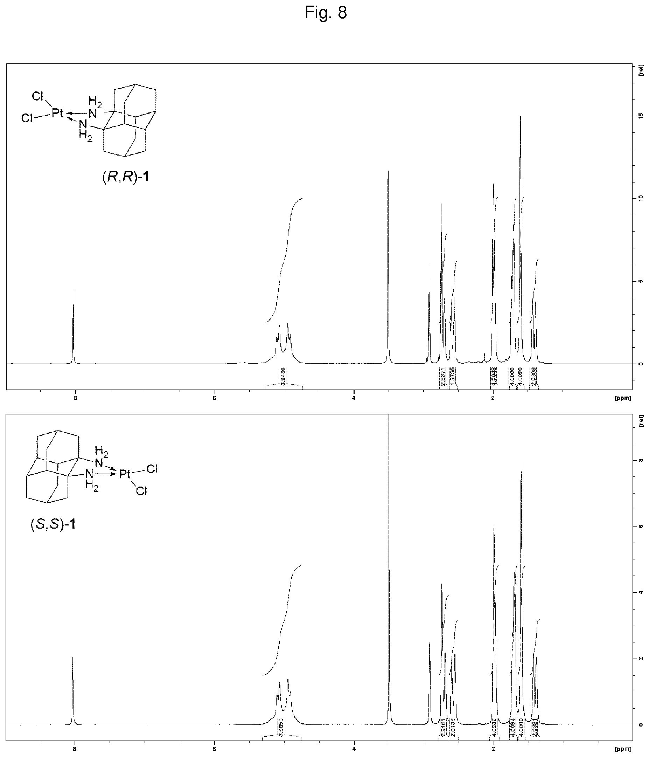

- Fig. 8:: ¹H NMR spectra of (R,R)-**1** and (S,S)-**1**.

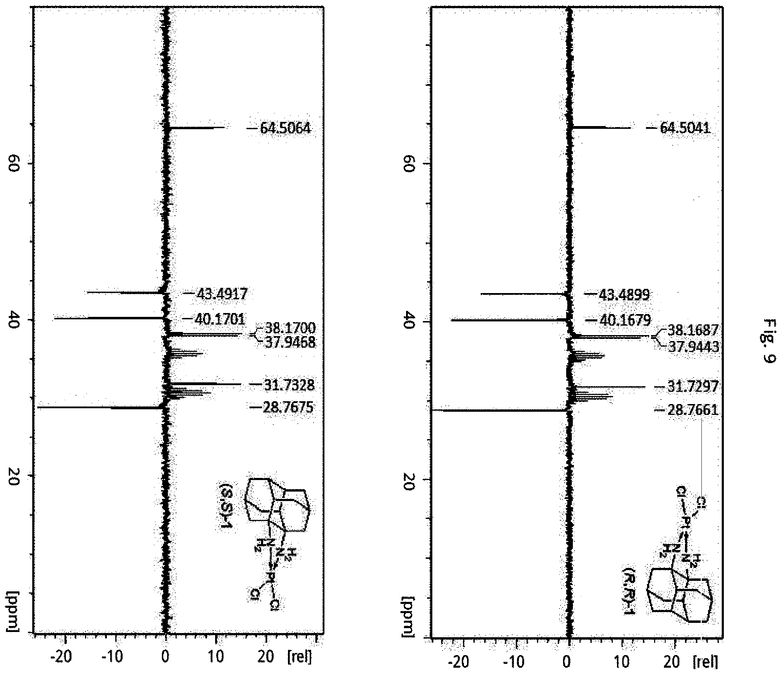

- Fig. 9:: ¹³C NMR spectra of (*R*,*R*)-**1** and (*S*,*S*)-**1**.

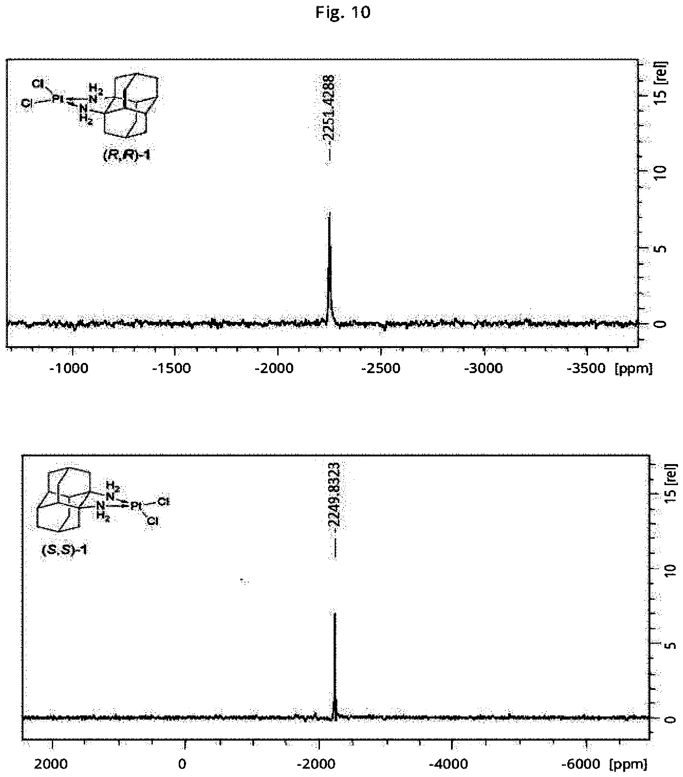

- Fig. 10:: ¹⁹⁵Pt NMR spectra of (*R*,*R*)-**1** and (*S*,*S*)-**1**.

## Claims

1. Compound according to formula **I**, 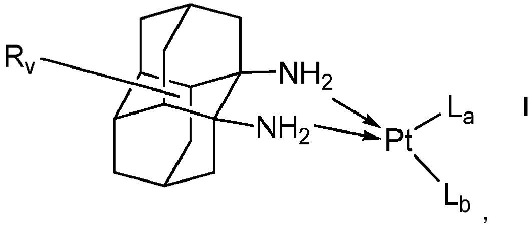 **characterized in that**
- the diamantane core-structure is substituted with at least one substituent Rᵥ, independently from each other chosen from the list comprising the substituents -H, -F, -Cl, -Br-, -I, methyl, ethyl, propyl, isopropyl, n-butyl, sec-butyl, tert-butyl, phenyl, -OH, -OMe, -OEt, -NH₂;
- the ligands Lₐ and L_{b} are independently chosen from the list comprising the ligands -F, -Cl, -Br, -I, -OH, -OR
or
the ligands Lₐ and L_{b} are together forming a bidentate/bivalent ligand of the type -X-(CH₂)s-Y- whereat
X and Y are independently from each other chosen from the substituents -CO₂H, -CNR(OH), -CS(OH) or their deprotonated forms, and the index s adopts a value of 0, 1, 2, 3 or 4.

2. Process for the production of a compound according to formula **I,** 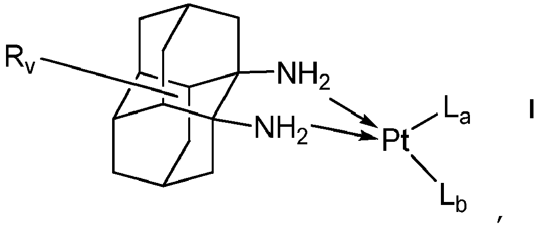 **characterized in that** the process comprises the following steps:
a) preparing a solution
i) of a salt of formula M₂PtX₄
or
ii) of a salt of formula M₂[Pt(L)₂]
whereat
M is at least one metal independently chosen from the alkali metals Li, Na, K, Rb, Cs
and
iii) X is at least one halogen, independently chosen from the list of halogens comprising F, Cl, Br, I
or
iv) L is a bidentate ligand of the type -X-(CH₂)s-Y-, whereat
X and Y are independently from each other chosen from the substituents -CO₂⁻-, -CNR(O)⁻, -CS(O)⁻,
and the index s adopts a value of 0, 1, 2, 3 or 4;
b) adding 1,2-diaminodiamantane or a solution of 1,2-diaminodiamantane to the solution according to step a) whereby a reaction mixture is formed;
c) stirring the reaction mixture of step b) whereby a precipitate is formed;
d) removing the precipitate from step c) and purifying it in order to get the pure compound according to formula **I.**

3. A medicament comprising a therapeutic effective amount of a compound according to claim 1 for use in the treatment of cancer of humans and/or mammals.
